# EUROPEAN PATENT SPECIFICATION

(11) **EP 0 186 505 B1**
(45) Date of publication and mention of the grant of the patent: **05.05.1993**
(21) Application number: 85309447.2
(22) Date of filing: 23.12.1985
(51) Int. Cl.: A61K 31/28

(54) **Pharmaceutical compositions containing organogermanium compound and their use**
Pharmazeutische Zusammensetzungen mit Gehalt an einer Organo-Germaniumverbindung und ihre Anwendung
Compositions pharmaceutiques contenant un composé organique du germanium et leur application

(30) Priority: 25.12.1984 JP 272057/84
(43) Date of publication of application: 02.07.1986
(73) Proprietor: SANWA KAGAKU KENKYUSHO CO., LTD., Higashi-ku Nagoya-shi Aichi-ken (JP)
(72) Inventor: Sawai, Kiichi, Funabashi-shi Chiba-ken (JP); Kurono, Masayasu, Nagoya-shi Aichi-ken (JP); Awaya, Juichi, Nagoya-shi Aichi-ken (JP); Kojima, Akio, Kasugai-shi Aichi-ken (JP); Ninomiya, Hideaki, Nagoya-shi Aichi-ken (JP); Ishiwata, Yoshiro, Nagoya-shi Aichi-ken (JP); Nakajima, Masahiro, Gifu-shi Gifu-ken (JP)
(74) Representative: Diamond, Bryan Clive

(56) References cited:
- EP-A- 0 016 444
- FR-A- 2 418 805
- GB-A- 1 365 997
- PATENT ABSTRACTS OF JAPAN, vol. 10, no. 43 (C-329)[2100], 20th February 1986
- The Merck Index, 10th ed., p. 625, no. 4242 "Gelatin"

## Description

The present invention relates to a composition containing an organogermanium compound which is pharmaceutically useful as an immunity-adjusting agent, and it is an object of the invention to stabilise this compound.

Organogermanium compounds represented by the general formula 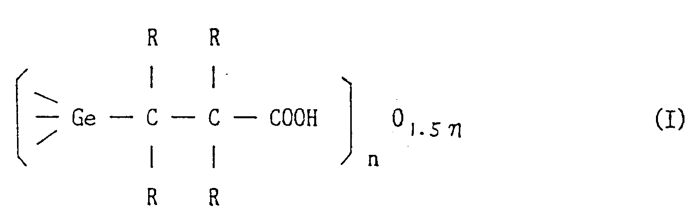
wherein n is an integer of 1 or more, R is hydrogen, alkyl, -COOH, -COOR', phenyl, 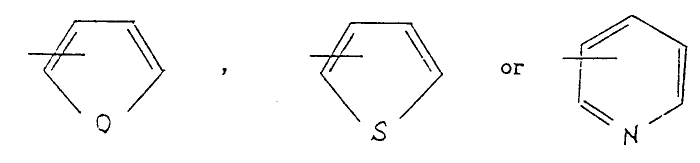
and R' is a lower alkyl group, have been the subject of great interest in recent years, due to their useful pharmacological activities, and thus various derivatives thereof have been synthesized.

However, these known organogermanium compounds have a common disadvantage of lack of stability to water. Namely, when such an organogermanium compound is prepared through hydrolysis of trichlorogermylpropionic acid, some different organogermanium compounds will be formed, as disclosed in Examined Jap. Pat. Appln. Gazette Nos. 2498/1971 and 53800/1982 as well as unexamined Jap. Pat. Appln. Gazette Nos. 102895/1982, i.e. the product will differ, due to a slight difference in the term or conditions of the hydrolysis reaction. Therefore, there is the possibility that one product may when suspended or dissolved in water change into another product, and this phenomenon has been reported (Examined Jap. Pat. Appln.Gazette Nos. 53800/1982 and 18399/1984).

The inventors have carefully studied the pharmacological activities of various organogermanium compounds represented by Formula I, which have been prepared by a common process but under different synthesis conditions and they have found that each compound shows a remarkable difference in extent of its pharmacological activity. Now, it is, of course, required to obtain specific organogermanium compounds which show a high and stable pharmacological activity, to utilize them as effective component of a pharmaceutical agent. However, it has further been confirmed by the inventors that for the compounds of Formula I, and more particularly those wherein some or all of the substituents R are hydrogen, the polymerization degree varies due to a slight difference in synthetic conditions therefor , that the form of the pharmaceutical agent is limited to a solid one only, since an inter-molecular bond therein is easily broken due to a slight change in environment or atmosphere, and that stable pharmacological acitivities inherent to the compound cannot be expected, since at least partial decomposition thereof occurs prior to its reaching a desired absorption area in a living body.

Hitherto, there have been a large number of published reports to the effect that such organogermanium compounds have an immunity accelerating action as one of their pharmacological actions but each of such compounds has not only been employed for developing a pharmaceutical agent, due to its low stability and other difficulties but also has been considered as a harmful substance to various diseases or disorders concerning an immunity acceleration system.

However, the inventors have now found that the organogermanium compounds have great effectivness on various immunity disorders, namely both the immunity inhibition system disorders and the immunity acceleration system disorders, and thus the "immunity accelerating action" which has hithereto been reported and widely been accepted is not correct and is in fact an immunity adjusting or regulating action.

Therefore, an object of the invention is to provide a composition, in which at least one of the compounds of Formula I is physico-chemically and pharmacologically stabilized.

Another object of the invention is to provide an immunity adjusting agent useful as a therapeutic agent for various immunity disorders due to an abnormal increase or decrease of immunity function in living bodies.

According to the present invention we provide a stabilized 3-oxygermylpropionic acid polymer composition, which comprises (a) 1 part by weight of 3-oxygermylpropionic acid polymer of the formula

[GeCH₂CH₂COOH]ₙO_{1.5n}

wherein n is an integer of 1 or more, and (b) 0.01 to 200 parts by weight of a high molecular weight substance as a stabilizer for the polymer and selected from cattle fetal serum, serum albumin, pepsin, polyvinylpyrrolidone, gelatin, protamine, hydroxypropylmethylcellulose, polypeptone, yeast extract, tryptone, tryptose, dextrose, refined sugar, starch and cellulose.

Said 3-oxygermylpropionic acid polymer can be obtained by treating germanium dioxide with hypophosphorous acid or a salt thereof in the presence of hydrochloric acid; reacting the resulting chlorogermanium-phosphoric acid complex with acrylic acid; dissolving the resulting 3-trichlorogermylpropionic acid in an organic solvent soluble in water; and then adding water to the solution, said polymer crystallizing as white needles with a decomposition temperature of 240°C.

GB-A-1365997 discloses a medicine for treating hypertension, comprising carboxyethyl germanium sesquioxide and a pharmacologically acceptable carrier. The carrier is not specifically described.

EP-A-0016444 discloses the use of the compound (Ge CH₂CH₂COOH)₂O₃ for inducing interferon production in living cells.

FR-A-2418805 (= GB-A-2018265) also discloses this compound but as a higher polymer; namely (GeCH₂CH₂COOZ)ₙO_{1.5n} in pharmaceutical preparation for a variety of uses, where n is 3 or more.

These publications do not disclose compositions of the oxygermylpropionic acid compound together with a stabilizer.

The stabilized composition of the invention can be admixed to make a desired pharmaceutical composition, with a filler, binder, disintegrator or the like additive which has no reactivity to the organogermanium polymer compound and no pharmacological activity to a delayed type of immunity response reaction test.

The form of the added pharmaceutical agent may freely be selected, so that the composition can be a solid, for instance a tablet, capsule, granules, fine grains, powder, dry syrup, or suppository, a solution in a solvent, for instance for injection or oral dosage or external lotion; or a semi-solid, for instance a cream or jelly for external application.

It is preferable to dose humans in an amount of 0.3 to 20 mg/kg, for instance 1 mg/kg, as the amount of the organogermanium compound.

By means of the invention, the organogermanium compound as the main component is effectively stabilized to allow said choice of its form, so that the pharmacological activities of the organogermanium compound can be appropriately utilized.

The composition of the invention shows a powerful and stable physiological activity on an immunity system and is useful as a curing agent for various immunity disorders such as a tumor, viral disease, phlegmasia, hepatopathy, nephropathy and collagenosis, as well as an inhibition agent to a negative reaction which can be caused in organ transplantation.

In the accompanying drawings:
Figs. 1-7 are graphs showing IR spectra measured on an organogermanium compound used in the invention, in which
Fig. 1 is on the compound alone,
Fig. 2 is on a composition of the compound and a serum albumin, which has been stored 30 days,
Fig. 3 is on a composition of the original compound and hydroxypropylcellulose, which has been stored 30 days,
Fig. 4 is on a composition of the compound and γ-globulin, which has been stored 30 days,
Fig. 5 is on a composition of the compound and pepsin, which has been stored 30 days,
Figs. 6 and 7 are on a 4% aqueous solution of the compound alone, which had been stored 24 and 60 hours, respectively;
Figs. 8a and 8b are graphs showing the influence of the compound on antibody production ability, when normal mice are sensitized with SRBC of 2 x 10⁸ and 2 x 10⁷, respectively;
Fig. 9 is a graph showing the influence of the compound on antibody production ability in mice affected with a cancer;
Figs. 10a and 10b are graphs showing the influence of the compound on antibody production ability in NZB/W F₁ mice and BALB/C mice, respectively;
Fig. 11 is a graph showing the influence of the compound on the positive Arthus reaction in guinea pigs; and
Fig. 12 is a graph showing the influence of the compound on adjuvant arthritis in rats.

The invention will now be explained with reference to stability test examples, pharmacological test examples and pharmaceutical agent preparation examples.

### Stability Tests

### 1) Physico-chemical stability test

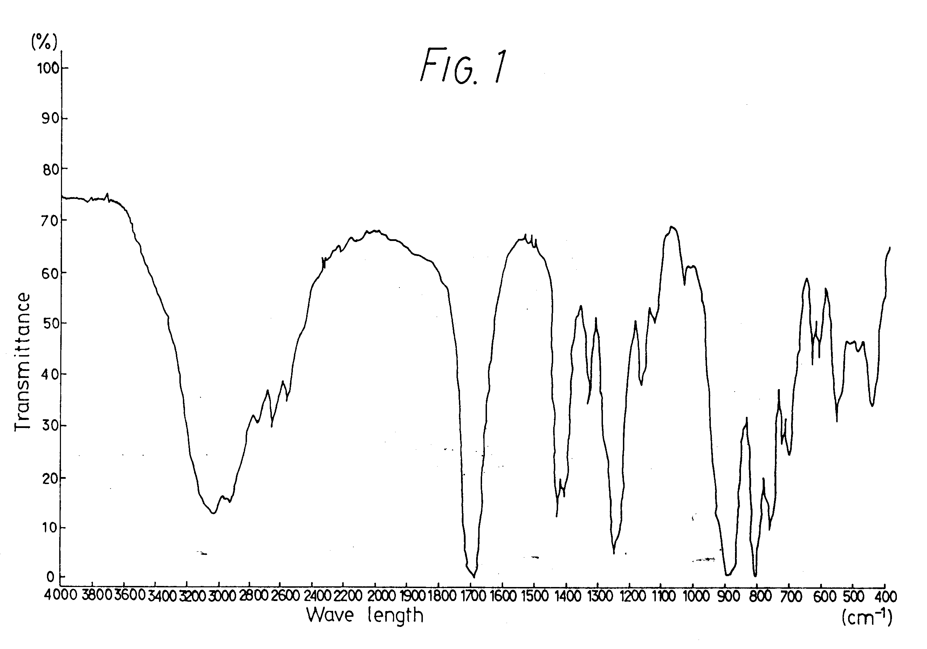

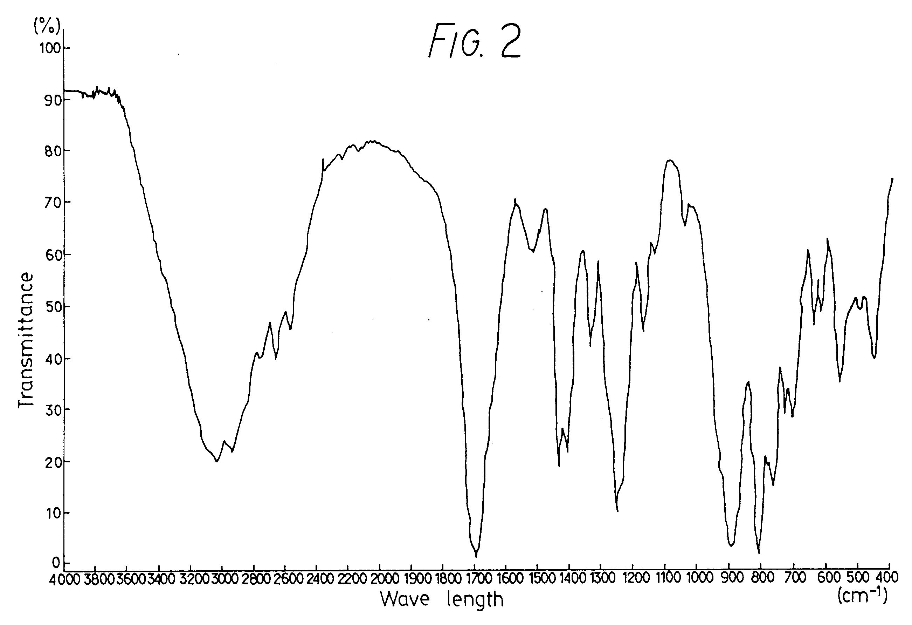

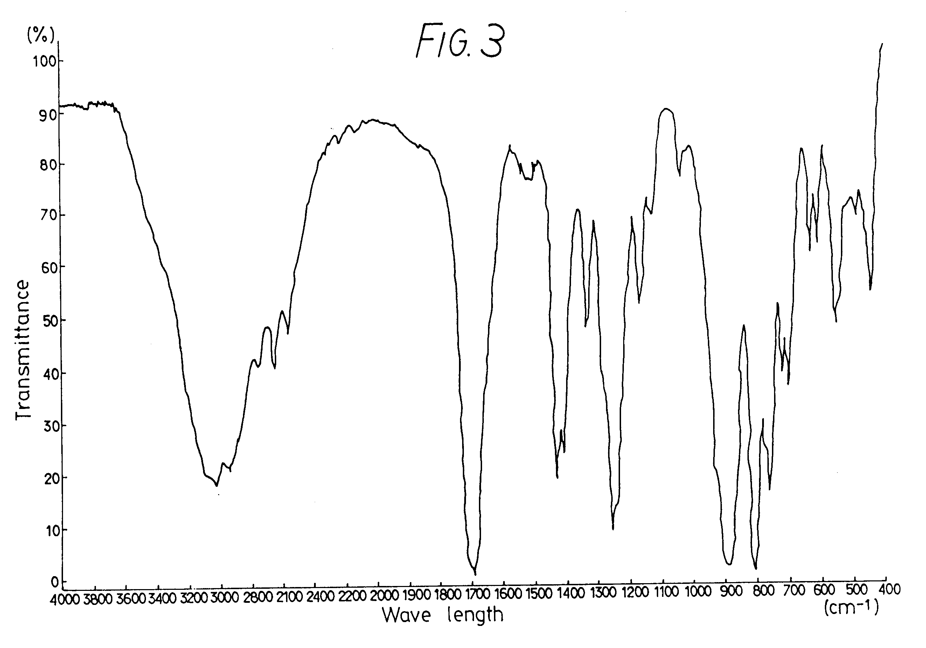

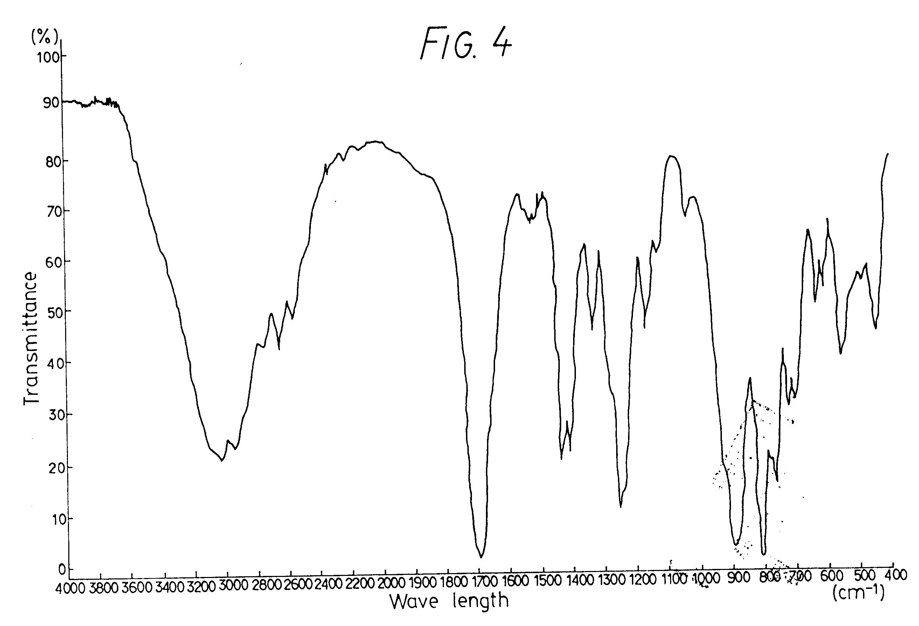

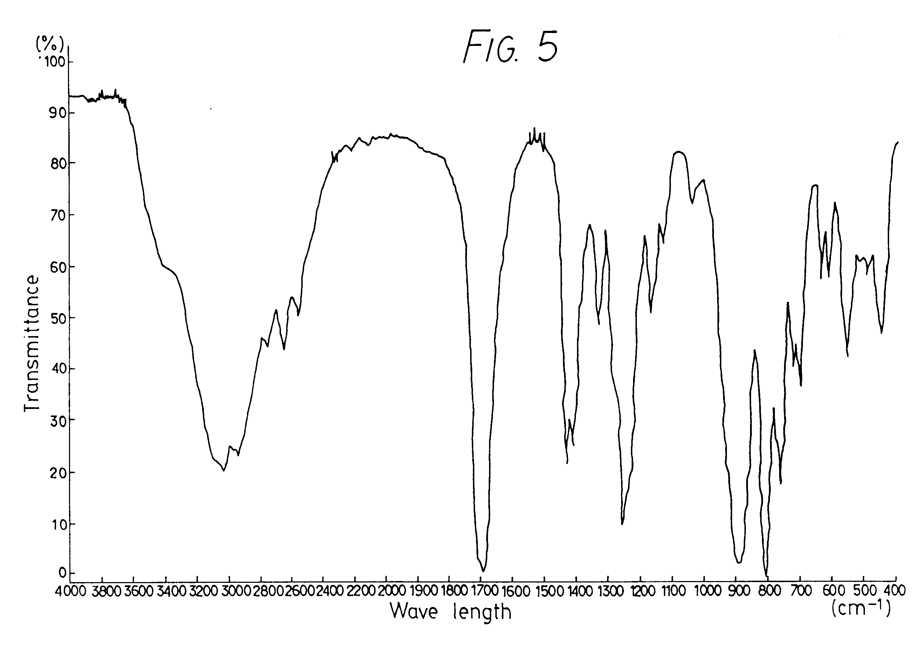

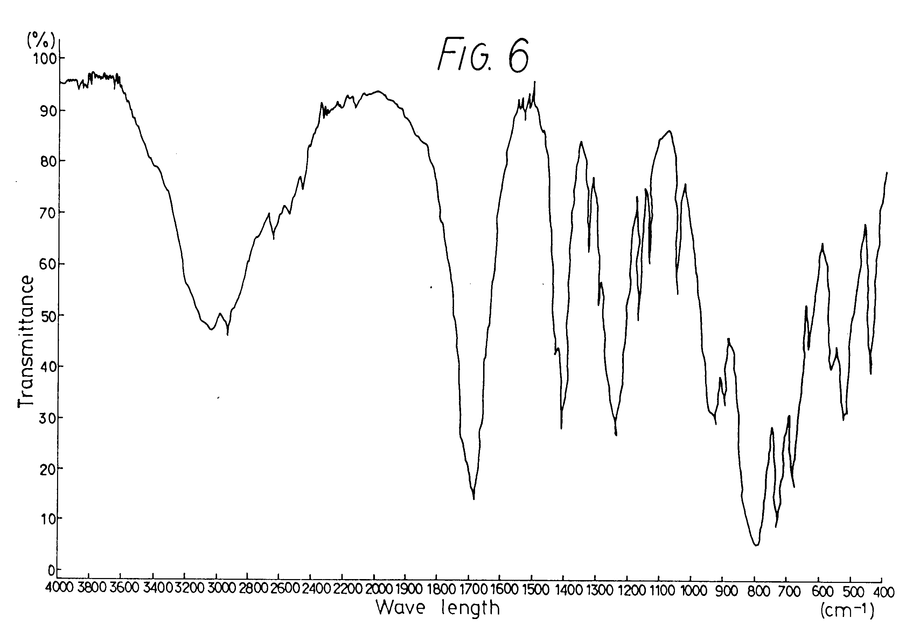

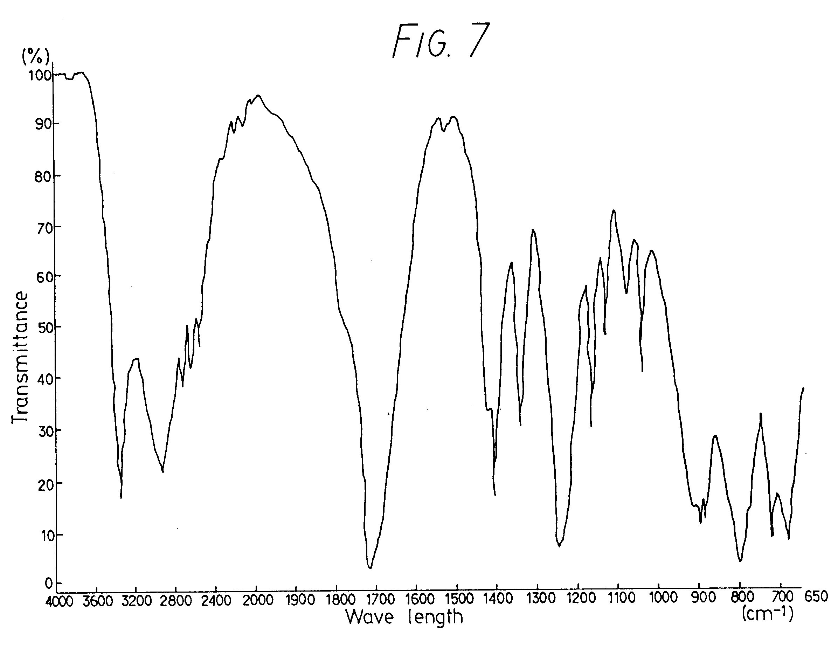

a) To 5 ml of 4% cattle serum albumin solution, 200 mg of the organogermanium compound were added and dispersed therein by a mixer to prepare a composition of the invention (4% suspension of the organogermanium compound).
   The suspension was stored in a thermostat kept at 25°C, sampled after periods of 1, 3, 9, 15 and 30 days and filtered. Each resulting solid substance was washed with acetone and ethanol and then dried for 1 hour at 105°C.
   The stability of the resulting dried substance was checked by measuring its IR spectrum, in accordance with the potassium bromide tablet method. An IR spectrum of the original or non-treated organogermanium compound is shown in Fig. 1 and has characteristic absorption peaks at 1695, 1435, 1255, 890 and 805 cm, and thus the stability was judged on the basis of such characteristic absorption spectrum.
   The tested composition was kept in a stable state, even when it had been stored for 30 days (see Fig. 2).
   As as control, a composition was prepared with use of water in lieu of the serum albumin and its stability was measured in a manner similar to the above to find that a disturbance in the absorption spectrum was first observed on the sample having been stored for 24 hours (see Fig. 6) and that the disturbance was further increased on the sample stored for 60 hours (see Fig. 7). This shows that a modification or decomposition occurs in the organogermanium compound in control composition.
b) Compositions according to the invention were prepared as in the case of said Item a but with use of hydroxypropylcellulose, γ-globulin and pepsin, respectively, in lieu of the serum albumin. Each of the compositions was tested as in the case of said Item a to find that the organogermanium compound is kept in a stable state, by virtue of coexistence with such a high molecular carrier (see Figs. 3 to 5).

A similar result was obtained on various compositions, wherein geratin, protamine, hydroxypropylmethylcellulose, polyvinylpyrrolidone, polyacryloamide, peptone, polypeptone, yeast extract, tryptone, tryptose, dextrose, lactose, refined sugar, glucose, starch or cellulose was employed as the high molecular carrier for the organogermanium compound, in lieu of the serum albumin.

### 2. Biological stability test

### Effect of delayed type hyperergy (DTH) on cancered mouse

After implantation of 10⁶ cells of sarcoma 180 cancer cell in an abdominal canal of ICR mice, 10⁶ corpuscles of sheep red blood corpuscle (SRBC) were venously injected for sensitization. After 4 days from the implantation, 2 x 10⁸ cells of SRBC were injected into a heel of a hind leg of said mice to cause the DTH. After 24 hours from the dosage, a degree of swelling was checked by measuring the thickness of the heel.

On the other hand, various testing compositions (Nos. 1 to 10 in following Table 1) were prepared with use of various high molecular carriers and adding the organogermanium compound, so as to make its concentration 1 mg/10 ml, and a control testing composition was prepared by adding the organogermanium compound to water to make its concentration 1 mg/10 ml. Each of the compositions was orally dosed to each mouse, respectively before 4 days of the cancer cell (Sarcoma 180) implantation and in an amount of 1 mg/10 ml/kg.

Results are shown in the following Table 1. From the Table, it can be seen that each of the compositions according to the invention increases the DTH of the cancered mouse but the control composition is not effective in the dosing amount of 1 mg/kg.

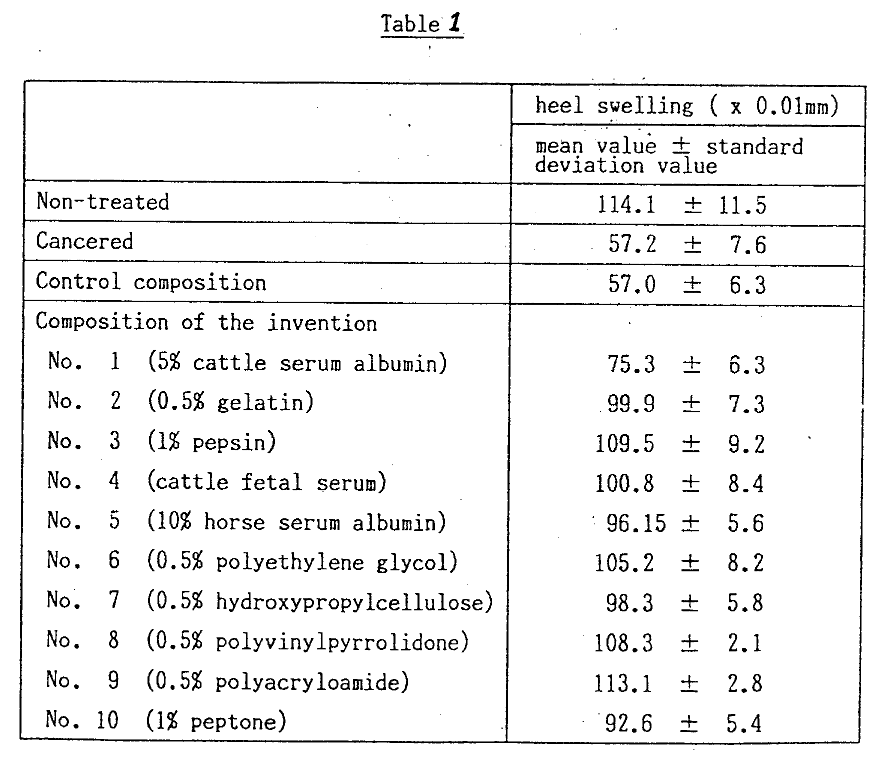

### Pharmacological Test Example 1

### (Influence of organogermanium compound on antibody production ability in normal mouse)

### a) Object

The effect of the organogermanium compound is checked by sensitizing mice with an antibody in an amount generating a sufficient antigen excitement to develop an immunity response in maximum level or not developing a sufficient immunity response due to no sufficient antigen excitement.

### b) Operation

To each group of ICR mice (age, 5 weeks), 2 x 10⁸ and 2 x 10⁷ corpuscles of sheep red blood corpuscle (SRBC) as an antibody were venously injected for sensitization in a tail vein of the mice. Then, immediately, the organogermanium compound dissolved in 4% cattle serum albumin solution was orally dosed to the sensitized mice in an amount of 0.1, 1.0 and 10 mg/kg, respectively. After 4 days from the sensitization, spleen cells were extracted to measure the number of PFC therein, which was used as an index of the antibody productivity.

### c) Results and consideration

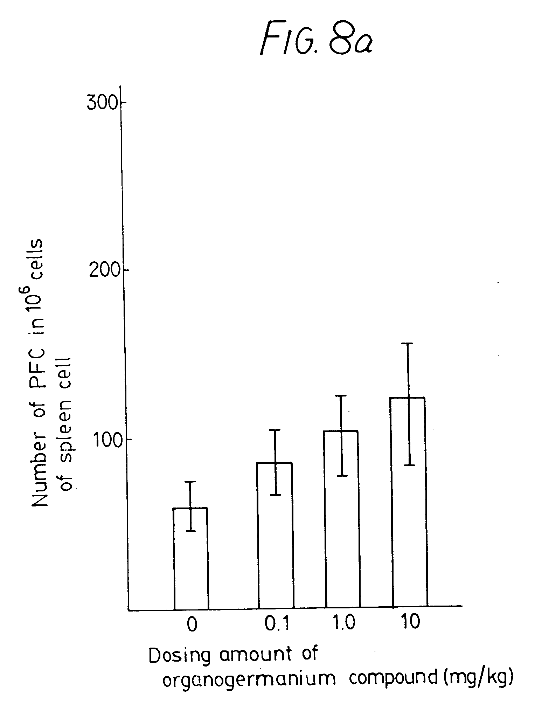

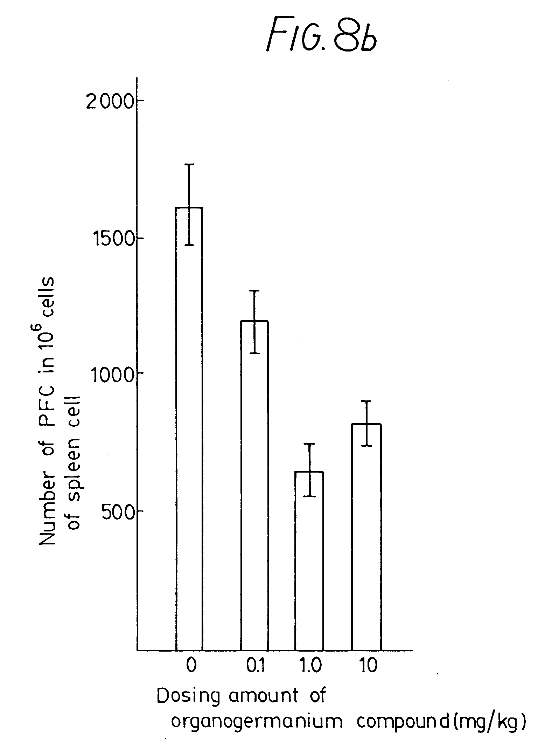

Results in the experimental group sensitized with 2 x 10⁸ corpuscles of SRBC and the other group sensitized with 2 x 10⁷ corpuscles of SRBC are shown in Figs. 8a and 8b, respectively. From the figures, the tendency can be seen that spleen cell PFC is decreased in the former group but is increased in the latter group.

These facts apparently show that the organogermanium compound has an immunity adjusting action.

### Pharmacological Test Example 2

### (Influence of organogermanium compound on antibody production ability in cancered mouse)

### a) Object

Similar to the object as referred to in the Pharmacological Test Example 1.

### b) Operation

2 x 10⁶ cells of a mouse tumor cell (Sarcoma 180) were implanted under a skin of a side part of ICR male mice to form a solid cancer. The organogermanium compound (Compound No. 1 in said Table 1) dissolved in 4% cattle serum albumin solution was orally dosed to the cancered mice for 5 days after lapsed 9 days from the implantation, 2 x 10⁹ corpuscles of SRBC were injected in a tail vein of the mice for sensitization. After 4 days from the sensitization, spleen cells were extracted to measure the PFC therein, which was used as an index of the antibody productivity.

### c) Results and consideration

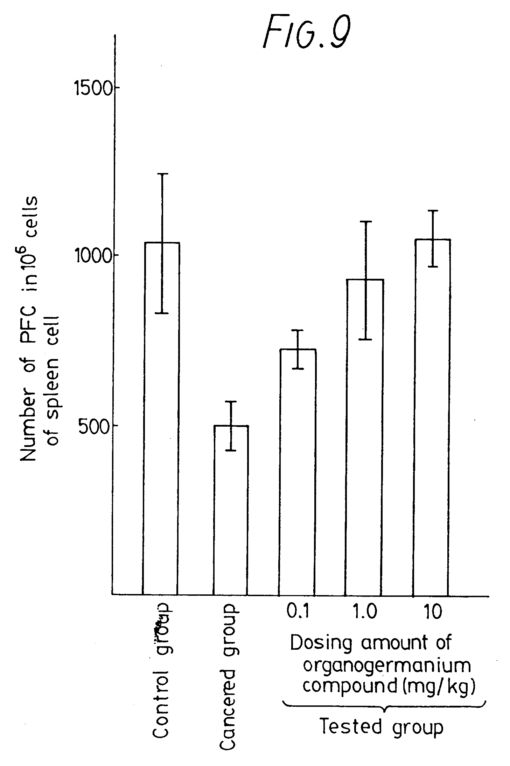

Results are shown in Fig. 9. As seen from the figure, it has been found that the antibody production ability reduces due to generation of the cancer, but by dosing the organogermanium compound, the antibody production ability recovers towards a normal level, depending on the dosage amount of the compound.

By taking this result and the result as shown in Fig. 8a into consideration, it is apparent that the organogermanium compound has an immunity adjusting action.

### Pharmacological Test Example 3

### (Influence of the organogermanium compound on antibody production ability in culture system for mouth lymphocytes)

### a) Object

Influence of the organogermanium compound on SRBC is checked on a culture system of lymphocytes extracted from NZB/W F₁ mice who generate a self immunity disease due to a functional reduction of suppresser T cells as well as normal BALB/C mice.

### b) Operation

Spleen lymphocytes were extracted from NZB/W F₁ male mice (age, 14 - 15 weeks) and BALB/C male mice (age, 10 - 13 weeks), washed with a Hanks solution, the lymphocytes were dispersed through a 100 mesh filter and then washed twice with the Hanks solution. The resulting lymphocytes were dispersed in a 10% cattle fatal serum added BPMI 1640 culture medium (including 2-mercaptoethanol in 5 x 10 ⁵M) and containing the organo-germanium compound and a lymphocyte concentration of the dispersion was regulated with a Turk solution into 1.2 x 10⁷ corpuscles/ml.

On the other hand, SRBC was washed twice with the Hanks solution and then dispersed in a manner similar to the above into the 10% cattle fetal serum added RPMI culture medium containing the organogermanium compound, and a concentration of the SRBR was regulated into 1.2 x 10⁷ corpuscles/ml.

Each 0.5 ml of said lymphocyte suspended medium and said SRBC suspended medium was sampled out and mixed. The mixture was charged onto a microplate and cultivated for 4 to 5 days at 37°C under 5% CO₂ condition. Thereafter, an anti SRBC antibody produced cell number was measured with a slide method, as a plaque forming cell number.

### c) Results and consideration

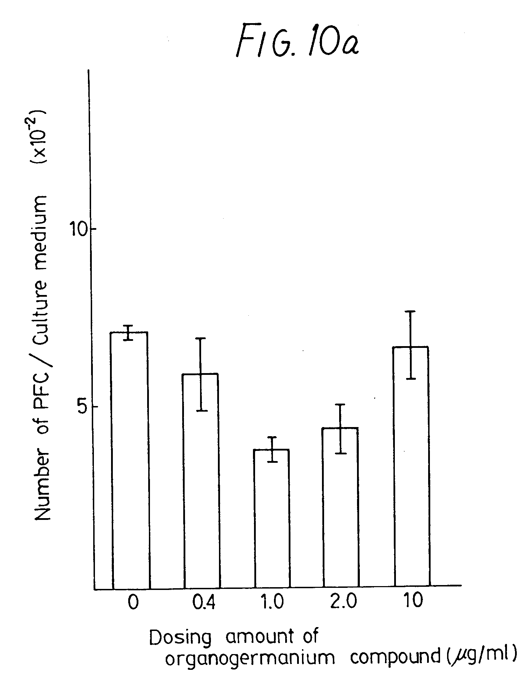

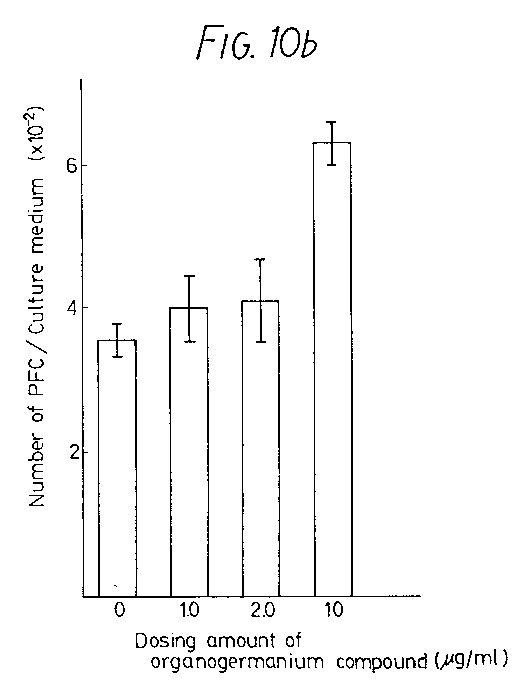

Results are shown in Figs. 10a and 10b. From the figures, an inhibition in antibody production ability is recognized on the NZB/W F₁ mouse lymphocytes, when the organogermanium compound is added in the amount of 1 to 2 µg/ml (Fig. 10a) but on the BALB/C mouse lymphocytes,no change can be recognized in antibody producing function by adding the organogermanium compound in such amount and on the contrary thereto, an acceleration of the function can be seen,when the compound is added in the amount of 10 µg/ml (Fig. 10b).

These results also show apparently that a pharmacological action of the organogermanium compound to the immunity system is an immunity adjusting one.

### Pharmacological Test Example 4

### (Action of organogermanium compound to positive Arthus reaction in guinea pig)

### a) Object

For studying usability of theorganogermanium compound to an allergic parietitis, the action thereof on an active Arthus reaction in guinea pig is checked.

### b) Operation

To 2% egg albumin solution, a same amount of Freund's complete adjuvant was added to prepare an emulsion. The emulsion was injected 4 times, once a week, to Hartley male guinea pigs in a heel, under the skin and in the femoralis muscle, for sensitization. After 10 days from the final sensitization, 0.1 ml of 1% egg albumin solution was injected under the skin of the back to measure the area of the resulting edema. The organogermanium compound was orally dosed for 30 days from the first sensitization in an amount of 0.1, 1.0 and 10 mg/kg/day.

### c) Results

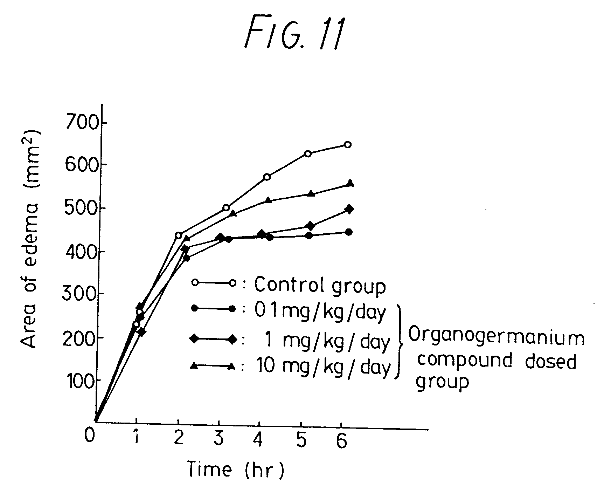

Each change in the adema area is shown in Fig. 11. From the figure, a recognizable inhibition of the phlegmasia can be found in the groups, to which the organogermanium compound was dosed in a ratio of 0.1 and 1 mg/kg/day, respectively.

### Pharmacological Test Example 5

### (Action of organogermanium compound to adjuvant arthritis)

### a) Object

An effect of the organogermanium compound on prevention of an adjuvant arthritis is checked.

### b) Operation

0.05 ml of an adjuvant (prepared by suspending 0.6 mg of micobacteriumbutircum into 0.05 ml of liquid paraffin) was injected under a skin of hind leg heel in S.D. male rats. After 1, 3, 5, 7, 21, and 28 days from the adjuvant dosage, a volume of the dosed and none-dosed legs was measured to determine a ratio of the edema.

The organogermanium compound was orally dosed for 28 days from the adjuvant dosage, in an amount of 1, 10 and 100 mg/kg/day, respectively.

### c) Results and consideration

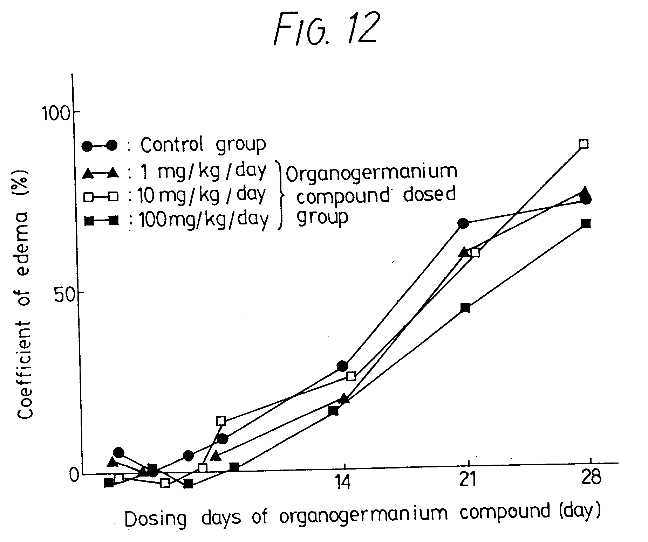

Results are shown in Fig. 12. From the figure, an inhibition effect to a secondary phlegmasia after 14 to 28 days can be recognized in the group, to which the organogermanium compound was dosed in the amount of 100 mg/kg/day.

### Pharmaceutical Agent Preparation Example 1

### (Injection)

To 0.1% solution of sodium carboxymethylcellulose, the organogermanium compound was added to make a concentration of the organogermanium compound to 1.5% and then mannite was added and dissolved, so that the concentration of the mannite was made 2%. The resulting solution was sterilized by a filtration method and filled into each vial by 2 ml, and freeze dried to prepare a powder for preparing an injection.

The powder can be dissolved into isotonic sodium chloride before use.

### Pharmaceutical Agent Preparation Example 2

### (Lotion for external application)

The organogermanium compound was added into 0.5% solution of polyvinylpyrrolidone and dissolved therein to make a concentration of the organogermanium compound to 0.1%.

This solution can directly be applied on skin or mucosa for a therapeutic purpose.

### Pharmaceutical Agent Preparation Example 3

### (Cream for external application)

The organogermanium compound was added into 4% solution of bovine serum albumin and dissolved therein to make a concentration of the organogermanium compound to 1.0%, and then the solution was freeze dried. This dry powder composition was mixed with excipients in a following prescription to prepare a cream agent (ointment).

| | |
|---|---|
| the powder composition | 0.5 (g) |
| diethyl sebacate | 8.0 |
| spermaceti | 5.0 |
| sodium polyoxyethyleneoleyletherphosphate | 6.0 |
| sodium benzoate | 0.5 |
| petrolatum | a sufficient quantity |
| | T̅o̅t̅a̅l̅ 1̅0̅0̅ (̅g̅)̅ |

### Pharmaceutical Agent Preparation Example 4

### (Suppository)

The dry composition in Pharmaceutical Agent Preparation Example 3 was dispersed in melted higher fatty acid glycerides in following amount ratio and suppositories were prepared from the dispersion, in a conventional method.

| | |
|---|---|
| The powder composition | 60 (mg) |
| fatty base (cacao butter) | 1640 |
| | 1̅7̅0̅0̅ (̅m̅g̅)̅/̅p̅i̅e̅c̅e̅ |

### Pharmaceutical Agent Preparation Example 5

### (Tablet)

The organogermanium compound was added and dissolved in 1% aqueous solution of pepsin, to make concentration of the organogermanium compound 1% and then the solution was freeze dried.

The freezed dry composition was mixed with excipients in the following prescription to prepare tablets in a conventional method.

| | |
|---|---|
| The powder composition | 60 (mg) |
| lactose | 90 |
| calcium carboxymethylcellulose | 7 |
| light anhydrous silicic acid | 1 |
| magnesium stearate | 7 |
| | 1̅6̅5̅ (̅m̅g̅)̅/̅t̅a̅b̅l̅e̅t̅ |

### Pharmaceutical Agent Preparation Example 6

### (Capsule)

The freeze-dried composition in the Pharmaceutical Agent Preparation Example 5 was mixed with other ingredients in the following prescription and this mixture was filled into each hard gelatin capsule, in a conventional method to prepare encapsulated agent.

| | |
|---|---|
| The powdered composition | 30 (mg) |
| lactose | 107 |
| hydroxypropylmethylcellulose | 2 |
| magnesium stearate | 1 |
| | 1̅4̅0̅ (̅m̅g̅)̅/̅c̅a̅p̅s̅u̅l̅e̅ |

### Utilization Example

The external cream agent obtained by the Pharmaceutical agent Preparation Example 3 was given to 20 volunteers who had a red swelling due to a sting by an insect, food allergy, pain or itching due to piles or the like local disease to use the same, when needed. After a certain time period, the volunteers were questioned with results shown in the following Table 2. As seen from the Table, almost all answered to the effect that the cream agent has a curing effect.

**Table 2**

| **Item** | | | | |
|---|---|---|---|---|
| | Improved | Getting improved | Indefinite | Getting worse |
| pain or itching | 1 | 15 | 3 | 1 |
| red swelling | 11 | 3 | 5 | 1 |

## Claims

1. A stabilized 3-oxygermylpropionic acid polymer composition, which comprises 1 part by weight of 3-oxygermylpropionic acid polymer of the formula
[GeCH₂CH₂COOH]ₙO_{1.5n}
wherein n is an integer of 1 or more, and 0.01 to 200 parts by weight of a high molecular weight substance as a stabilizer for the polymer and selected from cattle fetal serum, serum albumin, pepsin, polyvinylpyrrolidone, gelatin, protamine, hydroxypropylmethylcellulose, polypeptone, yeast extract, tryptone, tryptose, dextrose, refined sugar, starch and cellulose.

2. A composition as claimed in Claim 1, wherein said 3-oxygermylpropionic acid polymer is one obtained by treating germanium dioxide with hypophosphorous acid or a salt thereof in the presence of hydrochloric acid; reacting the resulting chlorogermanium-phosphoric acid complex with acrylic acid; dissolving the resulting 3-trichlorogermylpropionic acid in an organic solvent soluble in water; and then adding water to the solution, said polymer crystallizing as white needles with a decomposition temperature of 240°C.

3. A composition as claimed in Claim 1 or 2, wherein the amount of said high molecular weight stabilizer is 0.05 to 5 parts by weight.

4. A composition as claimed in Claim 3, wherein said organic solvent soluble in water is acetone.

5. Use of a composition as claimed in Claim 1, 2, 3 or 4 for the manufacture of a medicament to treat human immunity disorders including disorders of the immunity inhibition system and of the immunity acceleration system.

6. Use as claimed in Claim 5, for the manufacture of a medicament for internal administration for curing a tumor, viral disease, phlegmasia, hepatopathy, nephropathy or collagenosis.

7. Use as claimed in Claim 5, for the manufacture of a medicament for external administration for treatment of an insect sting, food allergy or piles.

## Patentansprüche

1. Ein stabilisiertes 3-oxygermyl-Propionsäure-Polymer Präparat, welches einen Gewichts-Teil 3-oxygermyl-Propionsäure-Polymer der Formel
[GeCH₂CH₂COOH]ₙO_{1.5n}
worin n eine ganze Zahl von 1 oder mehr bedeutet, sowie 0.01 bis 200 Gewichts-Teile einer hochmolekulargewichtigen Substanz als Stabilisator für das Polymer und ausgewählt aus, Serum aus Rinder-Fötus, Serum Albumin, Pepsin, Polyvinylpyrrolidon, Gelatine, Protamin, Hydroxkpropylmethyl-Zellulose, Polypepton, Hefe-Extrakt, Trypton, Tryptose, Dextrose, raffiniertem Zucker, Stärke und Zellulose, enthält.

2. Ein Präparat nach Anspruch 1, in welchem das 3-oxygermyl-Propionsäure-Polymer ein solches ist, das durch die Behandlung von Germanium mit hypophosphorige Säure oder einem Salz davon, unter Anwesenheit von Chlorwasserstoff-Säure erhalten wird; aus der Reaktion von Chlor-Germanium-Phosphorsäure Komplex mit Acrylsäure; Lösen der entstehenden 3-oxygermyl-Propionsäure in einem organischen Lösungsmittel, das in Wasser löslich ist; und dann Wasser zur Lösung zugegeben wird, wobei das Polymer als weisse Nadeln , mit einer Zersetzungstemperator von 240°C kristallisiert

3. Ein Präparat nach Anspruch 1 oder 2, in welchem der Anteil des Stabilisators mit hohem Molekulargewicht 0.05 bis 5 Gewichtsteile beträgt.

4. Ein Präparat nach Anspruch 3, in welchem das organische Lösungsmittel, das in Wasser löslich ist, Aceton ist.

5. Verwendung eines Präparats nach Anspruch 1, 2, 3 oder 4 zur Herstellung eines Medikaments zur Behandlung von Störungen des menschlichen Immunität, eingeschlossen Störungen des Immun-Inhibitions-Systems und des Immun-Akzelerations-Systems.

6. Verwendung nach Anspruch 5, zum Herstellen eines Medikaments zur innerlichen Anwendung und Einnahme, zur Behandlung eines Tumors, einer Virus-Krankheit, Phlegmasia, Hepatopathie, Nephropathie, oder Kollagenose.

7. Verwendung nach Anspruch 5, zur Herstellung eines Medikaments zur äusserlichen Verwendung zur Behandlung eines Insektenstichs, einer Nahrungsmittel-Allergie oder von Hämorrhoiden.

## Revendications

1. Composition stabilisée du polymère de l'acide 3-oxygermylpropionique qui comprend 1 partie en poids de polymère de l'acide 3-oxygermylpropionique de la formule
[GeCH₂CH₂COOH]ₙO_{1.5n}
dans laquelle n est un nombre entier égal ou supérieur à 1, et de 0,01 à 200 parties en poids d'une substance à haut poids moléculaire comme stabilisant pour le polymère, choisie parmi le sérum de foetus bovin, la sérumalbumine, la pepsine, la polyvinylpyrrolidone, la gélatine, la protamine, l'hydroxypropylméthylcellulose, la polypeptone, l'extrait de levure, la tryptone, le tryptose, le dextrose, le sucre raffiné, l'amidon et la cellulose.

2. Composition selon la revendication 1, dans laquelle ledit polymère de l'acide 3-oxygermylpropionique est obtenu en traitant du dioxyde de germanium par de l'acide hypophosphoreux ou un sel de celui-ci, en présence d'acide chlorhydrique; en faisant réagir le complexe chlorogermanium - acide phosphorique avec de l'acide acrylique; en dissolvant l'acide 3-trichlorogermylpropionique dans un solvant organique soluble dans l'eau; et ensuite en ajoutant de l'eau à la solution, ledit polymère cristallisant sous la forme d'aiguilles blanches ayant une température de décomposition de 240°C.

3. Composition selon la revendication 1 ou 2, dans laquelle la quantité dudit stabilisant à haut poids moléculaire est de 0,05 à 5 parties, en poids.

4. Composition selon la revendication 3, dans laquelle ledit solvant organique soluble dans l'eau est l'acétone.

5. Utilisation d'une composition selon la revendication 1, 2, 3 ou 4 pour la fabrication d'un médicament pour le traitement de désordres de l'immunité chez l'homme, en particulier les désordres du système inhibant l'immunité et du système accélérant l'immunité.

6. Utilisation selon la revendication 5, pour la fabrication d'un médicament pour une administration interne dans le but de guérir une tumeur, une maladie virale, une phlegmatia, une hépatopathie, une neuropathie ou une collagénose.

7. Utilisation selon la revendication 5, pour la fabrication d'un médicament pour un usage externe, dans le traitement de piqûres d'insectes, d'allergies alimentaires ou des hémorroïdes.
